# EUROPEAN PATENT SPECIFICATION

(11) **EP 4 039 210 B1**
(45) Date of publication and mention of the grant of the patent: **11.11.2026**
(21) Application number: 21165793.7
(22) Date of filing: 30.03.2021
(51) Int. Cl.: A61B 18/12

(54) **VIRTUALLY-SHORTED ELECTRODES FOR AN IRE PULSE GENERATOR**
VIRTUELL ÜBERBRÜCKTE ELEKTRODEN FÜR EINEN IRE-IMPULSGENERATOR
ÉLECTRODES VIRTUELLEMENT COURT-CIRCUITÉES POUR UN GÉNÉRATEUR D'IMPULSIONS IRE

(30) Priority: 03.02.2021 US 202117166678
(43) Date of publication of application: 10.08.2022
(73) Proprietor: Biosense Webster (Israel) Ltd., 2066717 Yokneam (IL)
(72) Inventor: ALTMANN, Andres Claudio, Yokneam (IL); SHAMIS, Yuri, Yokneam (IL); GOVARI, Assaf, Yokneam (IL)
(74) Representative: Carpmaels & Ransford LLP

(56) References cited:
- US-A1- 2020 078 089
- US-A1- 2020 138 506

## Description

### FIELD OF THE INVENTION

The present invention relates generally to medical devices, and particularly to devices for irreversible electroporation of physiological tissues.

### BACKGROUND

Irreversible electroporation (IRE) is a soft tissue ablation technique that applies short pulses of strong electrical fields to create permanent and hence lethal nanopores in the cell membrane, thus disrupting the cellular homeostasis (internal physical and chemical conditions). Cell death following IRE results from apoptosis (programmed cell death) and not necrosis (cell injury, which results in the destruction of a cell through the action of its own enzymes) as in all other thermal or radiation based ablation techniques. IRE is commonly used in tumor ablation in regions where precision and conservation of the extracellular matrix, blood flow and nerves are of importance.

United States Patent Application Publication No. 2010/0125315 describes a method and system of providing therapy to a patient implanted with an array of electrodes is provided. Electrical stimulation current is conveyed from at least two of the electrodes to at least one of the electrodes along at least two electrical paths through tissue of the patient, and the electrical stimulation current is shifted between the electrical paths by actively adjusting one or more finite resistances respectively associated with one or more of the electrical paths.

US2020/0138506A1 discusses methods and systems for monitoring and modifying pulsed field ablation (PFA) energy delivery to prevent patient safety risks and/or delivery device failure. In particular, some methods and systems of US2020/0138506A1 are for detecting and preventing arcs and arc-induced plasma, and their causal events, during delivery of pulsed field ablation energy, as well as methods and systems for identifying conditions leading to potential delivery device failure and correcting charge imbalance or asymmetry.

### SUMMARY

Embodiments of the present invention that are described hereinbelow provide improved apparatus for irreversible electroporation of body tissues.

The invention is defined by the independent claims with further embodiments defined by the dependent claims.

### BRIEF DESCRIPTION OF THE DRAWINGS

The present invention will be more fully understood from the following detailed description of the embodiments thereof, taken together with the drawings in which:
Fig. 1 is a schematic pictorial illustration of a medical apparatus in the course of an IRE procedure, in accordance with an exemplary embodiment of the present invention;
Fig. 2 is a schematic illustration of a biphasic IRE pulse, in accordance with an exemplary embodiment of the present invention;
Fig. 3 is a schematic illustration of a burst of biphasic pulses, in accordance with an exemplary embodiment of the present invention;
Fig. 4 is a block diagram that schematically shows the connections between an IRE pulse generator, a controller, electrodes, and a return patch, in accordance with an exemplary embodiment of the present invention;
Fig. 5 is an electrical schematic diagram of a pulse routing and metrology assembly of Fig. 4, in accordance with an exemplary embodiment of the present invention; and
Fig. 6 is an electrical schematic diagram of two adjacent modules of the pulse routing and metrology assembly configured for IRE in bipolar mode, in accordance with an exemplary embodiment of the present invention.

### DETAILED DESCRIPTION OF EMBODIMENTS

### OVERVIEW

IRE is a predominantly non-thermal ablation process, which causes an increase of the tissue temperature by, at most, a few degrees for a few milliseconds. It thus differs from RF (radio frequency) ablation, which raises the tissue temperature by between 20 and 70°C and destroys cells through heating. IRE utilizes biphasic pulses (combinations of positive and negative pulses) in order to avoid muscle contraction due to a nonzero DC voltage component. Biphasic pulses are also commonly called "bipolar" pulses. However, as further detailed below, IRE may be carried out either in a unipolar mode or bipolar mode. In order to avoid confusion, the term "bipolar" is hereinbelow used only in the context of a bipolar mode of IRE.

Some IRE procedures use a balloon catheter, which has a balloon at its distal end and electrodes arrayed around the surface of the balloon. The balloon is inflated within the body cavity, and the electrodes are then brought into contact with the tissue that is to be electroporated. For electroporating tissue in small cavities within the body, for example in the left atrium of the heart, balloons of small diameter can be used, for example with a diameter less than 15 mm.

IRE may be carried out in either a bipolar mode, in which the electroporation currents flow from one electroporation electrode to another on the same catheter, or a unipolar mode, in which the electroporation currents flow between the electroporation electrodes on the catheter and an external electrode, referred to as a "return patch." The return patch is typically fixed to the body surface of the subject, for example on the skin of the subject's torso, with an electrical return connection to the IRE signal generator.

The electrodes arrayed on small-diameter balloons, as well as on other sorts of electrode assemblies used in IRE procedures, are commonly small in size, to enable precise targeting of the ablation energy. Typically, the electrical signal generator that applies the IRE pulses to the electrodes (also referred to herein as an "IRE pulse generator") enables individual activation of each of the electrodes via a respective channel of the generator. Because of their small size, each electrode touches and ablates only a small area of the tissue.

In some cases, it may be desirable to apply IRE over a larger area of the tissue than can be covered by a single small electrode. For this purpose, two or more adjacent electrodes can be grouped together by electrically shorting them to each other, thus generating a larger effective electroporation area. Implementation of this approach in hardware, however, requires additional high-voltage switches between the individual output channels of the IRE pulse generator. These additional switches are costly and require dedicated control lines, and they are limited in the flexibility that they can afford for creating different groupings of electrodes.

The embodiments of the present invention that are described herein address this problem by enabling "virtual shorting" of groups of two or more electrodes in the IRE system. The electrodes are "virtually shorted" in the sense that all the electrodes in the group apply the same voltage waveform, with the same amplitude and phase, simultaneously to the tissue with which they are in contact. Thus, the IRE current flows through a larger area of the tissue, defined by the contact locations of all the electrodes in the group.

This sort of virtual shorting, however, cannot be reliably accomplished simply by setting the IRE pulse generator to apply the same waveform to all of the electrodes in the group. For example, local differences in the impedance of the tissue, as well as differences in the contact impedance between the electrodes in the group and the tissue that they contact, can result in difference in the amplitude and phase of the IRE waveforms that are actually applied to the tissue by the different electrodes. This non-uniformity in the waveforms may cause the IRE currents to flow along unexpected paths through the tissue and give rise to unsatisfactory ablation effects.

Embodiments of the present invention that are described herein address this problem by measuring time-varying voltage differences between the electrodes in the group. Based on these measurements, the IRE pulse generator adjusts the biphasic electrical pulses applied to the electrodes in the group so that the voltage differences do not exceed a predetermined threshold at any time during application of the biphasic electrical pulses.

In the disclosed embodiments, in a unipolar IRE procedure the IRE pulse generator applies biphasic IRE pulses simultaneously to a selected group of electrodes on the probe, with an energy sufficient to electroporate the tissue contacted by the electrodes. A controller measures the time-varying voltage differences between the electrodes in the group and adjusts the amplitudes and phases of the IRE pulses so that the voltage differences do not exceed a predetermined threshold at any time during the application of the IRE pulses. This approach ensures that the group of electrodes applies the IRE pulses as if the group were a single large-area electrode.

In a bipolar IRE procedure, the IRE pulses are required to flow through the tissue from one group of electrodes to another group of electrodes. For this purpose, the controller adjusts the relative amplitudes and phases between the IRE pulses applied to the two groups so as to generate a train of IRE pulses between the two groups for bipolar electroporation in the tissue between these two groups of electrodes. Additionally, and similarly to unipolar IRE, the controller adjusts the amplitudes and phases of the IRE pulses within each group so that the voltage differences between the electrodes of the group do not exceed a predetermined threshold at any time during the application of the IRE pulses.

### SYSTEM DESCRIPTION

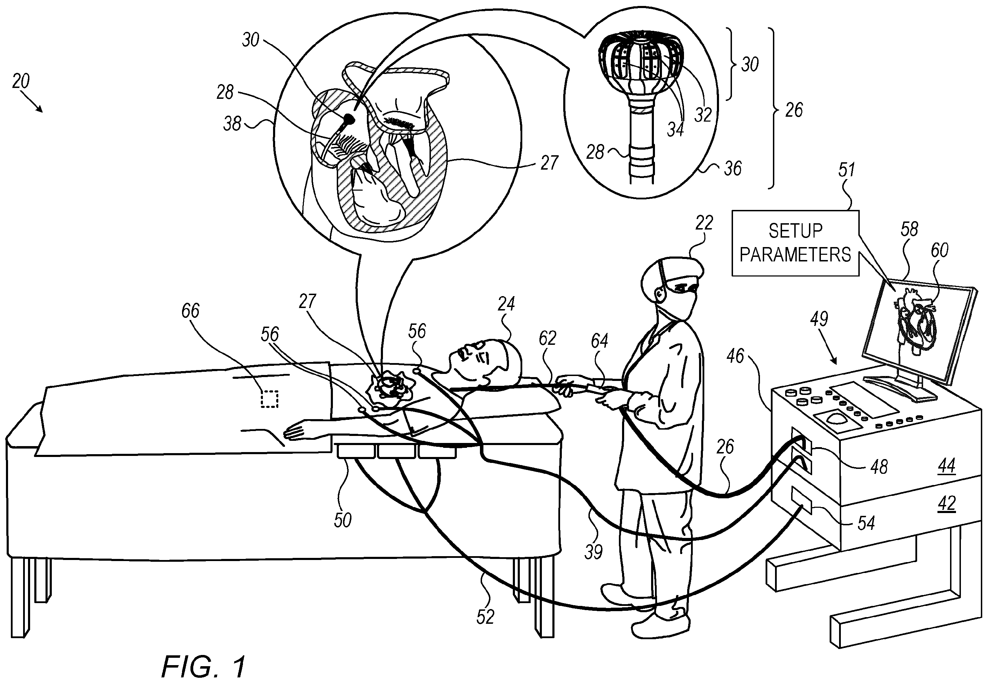

Fig. 1 is a schematic pictorial illustration of a medical apparatus 20 in the course of an IRE procedure, in accordance with an embodiment of the invention. A physician 22 performs the IRE procedure on a patient 24, using an electroporation catheter 26, with further details of the catheter described hereinbelow. The embodiment shown in the figures refers to an example of an IRE procedure in a chamber of a heart 27 using a balloon 32. In alternative embodiments, the IRE procedure may be performed using other types of catheters having multiple electrodes and may be performed not only in heart 27, but also in other organs and tissue, as will be apparent to those skilled in the art after reading the present description.

As shown in an inset 36, electroporation catheter 26 comprises a shaft 28 and a distal assembly 30, wherein the shaft functions as an insertion tube for inserting the distal assembly into a body cavity of patient 24, in this case into the chamber of heart 27. Distal assembly 30 comprises balloon 32 with a plurality of electroporation electrodes 34. Distal assembly 30 and a part of shaft 28 are also shown in an inset 38. In alternative embodiments, distal assembly 30 may comprise a structure different from a balloon.

Medical apparatus 20 further comprises a controller 42 and an electrical signal generator, configured as an IRE pulse generator 44, typically residing in a console 46; the controller and the signal generator may each comprise one or several circuit components. Further details of a signal generator of this sort are described in U.S. Patent Application No. 16/701,989, filed December 3, 2019, and in U.S. Patent Application No. 17/092,662, filed November 9, 2020. Catheter 26 is connected to console 46 via an electrical interface 48, such as a port or socket, through which IRE pulses are carried from IRE pulse generator 44 to distal assembly 30. Console 40 comprises input devices 49, such as a keyboard and a mouse, as well as a display screen 58.

Controller 42 receives from physician 22 (or another operator), prior to and/or during the electroporation procedure, setup parameters 51 for the procedure. For example, using one or more suitable input devices, such as a keyboard, mouse, or touch screen (not shown), physician 22 defines the electrical and temporal parameters of the IRE pulses to be applied to selected electrodes 34. Controller 42 passes suitable control signals to IRE pulse generator 44 for performing the IRE.

Controller 42 may be further configured to track the respective positions of electrodes 34 during the IRE procedure, using any suitable tracking technique. For example, distal assembly 30 may comprise one or more electromagnetic position sensors (not shown), which, in the presence of an external magnetic field generated by one or more magnetic-field generators 50, output signals that vary with the positions of the sensors. Based on these signals, controller 42 may ascertain the positions of electrodes 34. Magnetic-field generators 50 are connected to console 46 via cables 52 and an interface 54. Alternatively, for each electrode 34, controller 42 may ascertain the respective impedances between the electrode and multiple external electrodes 56, which are coupled to patient 24 at various different locations and are connected to console 46 by a cable 39. Controller 42 computes the ratios between these impedances, these ratios being indicative of the location of each electrode 34. As yet another alternative, the controller may use both electromagnetic tracking and impedance-based tracking, as described, for example, in US Patent No. 8,456, 182.

In some embodiments, controller 42 displays, on display screen 58, a relevant image 60 of the subject's anatomy, annotated, for example, to show the current position and orientation of distal assembly 30.

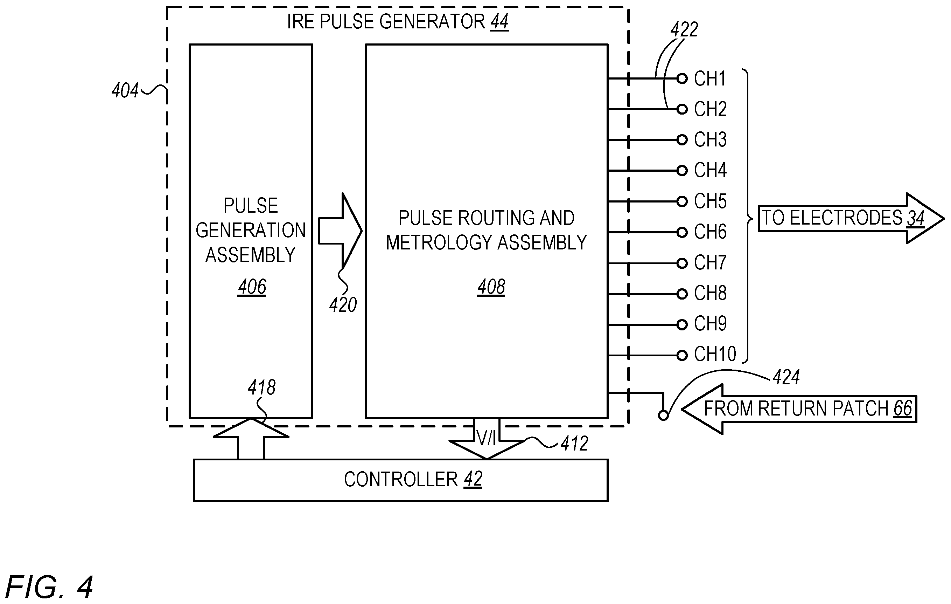

Controller 42 and electric IRE pulse generator 44 may typically comprise both analog and digital elements. Thus, controller 42 comprises an analog front-end with multiple inputs with respective analog-to-digital converters (ADCs) for monitoring the IRE pulses applied by IRE pulse generator 44 to each of electrodes 34. Controller 42 further comprises multiple digital output circuits for sending commands to IRE pulse generator 44 for adjusting the IRE pulses, as detailed in Figs. 4-6 hereinbelow.

Electric IRE pulse generator 44 typically comprises analog circuits for generating and amplifying the IRE pulses for electroporation, as well as digital input circuits for receiving digital control signals from controller 42.

Alternatively, the control signals may be passed from controller 42 to electric IRE pulse generator 44 in an analog form, provided that the controller and the IRE pulse generator are configured accordingly.

Typically, the functionality of controller 42, as described herein, is implemented at least partly in software. For example, controller 42 may comprise a programmed digital computing device comprising at least a central processing unit (CPU) and random access memory (RAM). Program code, including software programs, and/or data are loaded into the RAM for execution and processing by the CPU. The program code and/or data may be downloaded to the controller in electronic form, over a network, for example. Alternatively or additionally, the program code and/or data may be provided and/or stored on non-transitory tangible media, such as magnetic, optical, or electronic memory. Such program code and/or data, when provided to the controller, produce a machine or special-purpose computer, configured to perform the tasks described herein.

At the start of the IRE procedure, physician 22 inserts catheter 26 through a sheath 62 with balloon 32 in a collapsed configuration, and only after the catheter exits the sheath is the balloon inflated to its intended functional shape with a fluid that flows into the balloon through shaft 28. This functional shape is shown in insets 36 and 38. By containing balloon 32 in a collapsed configuration, sheath 62 also serves to minimize vascular trauma while the balloon is brought to the target location. Physician 22 navigates catheter 26 to a target location in heart 27 of patient 24, by manipulating the catheter, using a manipulator 64 near the proximal end of the catheter, and/or deflection from sheath 62. Physician 22 brings distal assembly 30 into contact with tissue, such as myocardial tissue, of heart 27. Next, under the control of physician 22 and controller 42, IRE pulse generator 44 generates IRE pulses, which are carried through catheter 26 over different respective channels, to electroporation electrodes 34.

In the unipolar mode of IRE, the electroporation currents flow from one or more electroporation electrodes 34 to an external electrode, or a "return patch" 66, which is coupled externally between patient 24, typically on the skin of the subject's torso, and IRE pulse generator 44. Catheters 26 with balloon 32 having a diameter less than 15 mm are often used for electroporating tissue in small cavities within the body, for example in the left atrium of heart 27. Due to the small size of electroporation electrodes 34 of these small-diameter balloons, energizing only one of the electrodes with IRE pulses may cause the electroporation to take place in too small of an area. Shorting several electrodes 34 in a group together would create an effectively larger area of electroporation. While this could be accomplished by adding shorting switches between the individual output channels of IRE pulse generator 44, such an addition is costly. In the disclosed embodiment, at least two electrodes 34 are grouped together and effectively shorted by adjusting the amplitudes and phases of the IRE pulses at each of these electrodes to be the same. For this purpose, controller 42 monitors the amplitudes and phases of the IRE pulses at each of electrodes 34 in the group, and sends control signals to IRE pulse generator 44 so as to equalize these amplitudes and phases.

In the bipolar mode of IRE, the electroporation currents flow between two electrodes or groups of electrodes 34, requiring a train of IRE pulses between the electrodes. In the disclosed embodiment, controller 42 monitors - as in the above described embodiment of unipolar IRE - the amplitudes and phases of the IRE pulses at each of electrodes 34, but now adjusts them so as to generate the required train of IRE pulses between the two electrodes or two groups of electrodes. Controller 42 further, similarly to unipolar IRE, equalizes the amplitudes and phases of the IRE pulses within each group.

Further details of IRE pulse generator 44 and controller 42 are shown in Figs. 4-6, below.

Notwithstanding the particular type of electroporation procedure illustrated in Fig. 1, it is noted that the embodiments described herein may be applied to any suitable type of multi-channel IRE procedure.

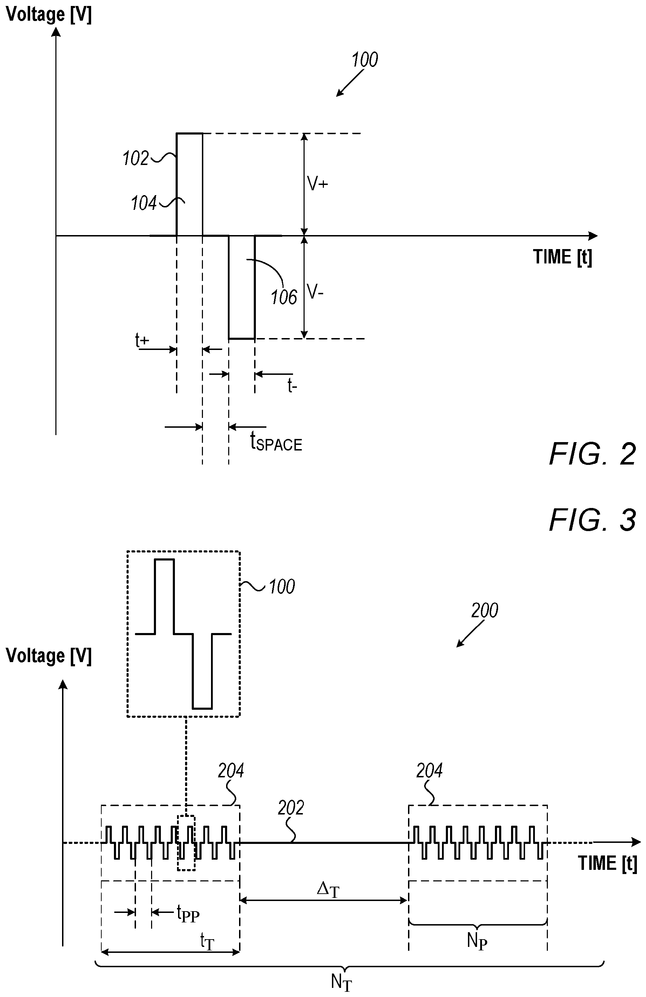

Fig. 2 is a schematic illustration of a biphasic IRE pulse 100, in accordance with an embodiment of the invention.

A curve 102 depicts the voltage V of biphasic IRE pulse 100 as a function of time t in an IRE procedure. The biphasic IRE pulse comprises a positive pulse 104 and a negative pulse 106, wherein the terms "positive" and "negative" refer to an arbitrarily chosen polarity of the two electrodes between which the biphasic pulse is applied. In unipolar IRE, the biphasic pulse may be applied either between a single electrode 34 and return patch 66 or between a group of electrodes 34 and return patch 66. For bipolar IRE, the biphasic pulse may be applied either between two electrodes 34 or between two groups of electrodes 34. The amplitude of positive pulse 104 is labeled as V+, and the temporal width of the pulse is labeled as t+. Similarly, the amplitude of negative pulse 106 is labeled as V-, and the temporal width of the pulse is labeled as t-. The temporal width between positive pulse 104 and negative pulse 106 is labeled as t_{SPACE}. Typical values for the parameters of biphasic pulse 100 are given in Table 1, below.

Fig. 3 is a schematic illustration of a burst 200 of biphasic pulses, in accordance with an embodiment of the invention.

In an IRE procedure, the IRE signals are delivered to electrodes 34 as one or more bursts 200, depicted by a curve 202. Burst 200 comprises N_{T} pulse trains 204, wherein each train comprises N_{P} biphasic pulses 100. The length of pulse train 204 is labeled as t_{T}. The period of biphasic pulses 100 within a pulse train 204 is labeled as t_{PP}, and the interval between consecutive trains is labeled as Δ_{T}, during which the signals are not applied. Typical values for the parameters of burst 200 are given in Table 1, below.

**Table 1: Typical values for the parameters of IRE signals**

| Parameter | Symbol | Typical values |
|---|---|---|
| Pulse amplitudes | V+, V- | 500-2000 V |
| Pulse widths | t+, t- | 0.5-5 µs |
| Spacing between positive and negative pulse | t_{SPACE} | 0.1-5 µs (1-10 ms when an optional RF signal is inserted between the positive and negative pulses) |
| Period of biphasic pulses in a pulse train | t_{PP} | 1-20 µs |
| Length of pulse train | t_{T} | 1- 100 µs |
| Number of biphasic pulses in a pulse train | N_{P} | 1-100 |
| Spacing between consecutive pulse trains | Δ_{T} | 0.3-1000 ms |
| Number of pulse trains in a burst | N_{T} | 1-100 |
| Length of a burst | | 0-500 ms |
| Energy per channel | | ≤60 J |
| Total time for IRE signal delivery | | ≤10 s |

Fig. 4 is a block diagram that schematically shows details of system 20 (Fig. 1), including the connections between IRE pulse generator 44, controller 42, electrodes 34 and return patch 66, in accordance with an embodiment of the invention.

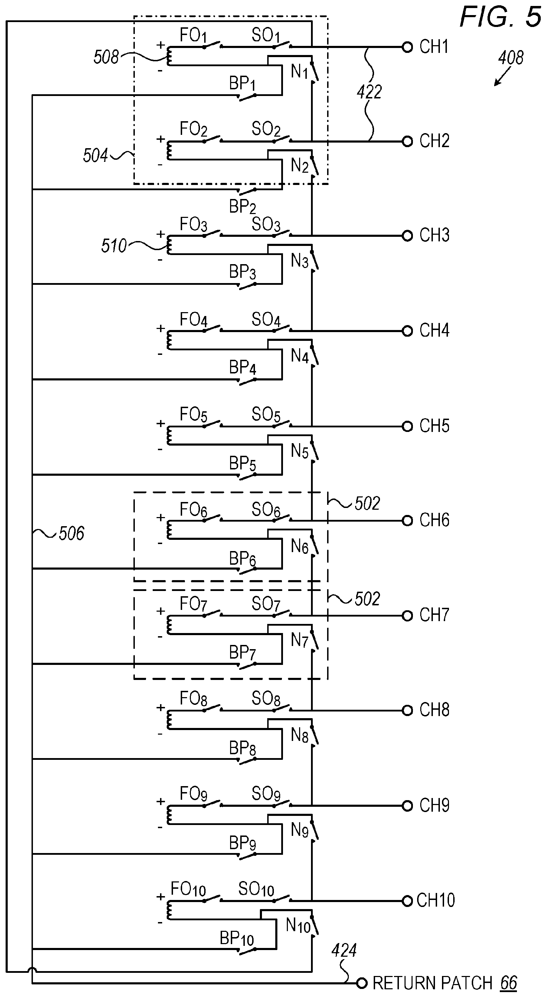

IRE pulse generator 44, delineated by a dotted-line frame 404, comprises a pulse generation assembly 406 and a pulse routing and metrology assembly 408, with the routing and metrology assembly further detailed in Figs. 5-6, below.

Controller 42 receives digital voltage and current signals 412 from pulse routing and metrology assembly 408, and communicates to pulse generation assembly 406 digital command signals 418, derived from setup parameters 51, commanding IRE pulse generator 44 to generate IRE pulses, such as those shown in Figs. 2-3, above. These IRE pulses are sent to pulse routing and metrology assembly 408 as analog pulse signals 420. Pulse routing and metrology assembly 408 is coupled to electrodes 34 through output channels 422, as well as to return patch 66 through connection 424. Fig. 4 shows ten output channels 422, labelled CH1-CH10. In the following description, a specific electrode 34 is called by the name of the specific channel coupled to it; for example electrode CH5 relates to the electrode that is coupled to CH5 of channels 422. Although Fig. 4 refers to ten channels 422, IRE pulse generator 44 may alternatively comprise a different number of channels, for example 8, 16, or 20 channels, or any other suitable number of channels.

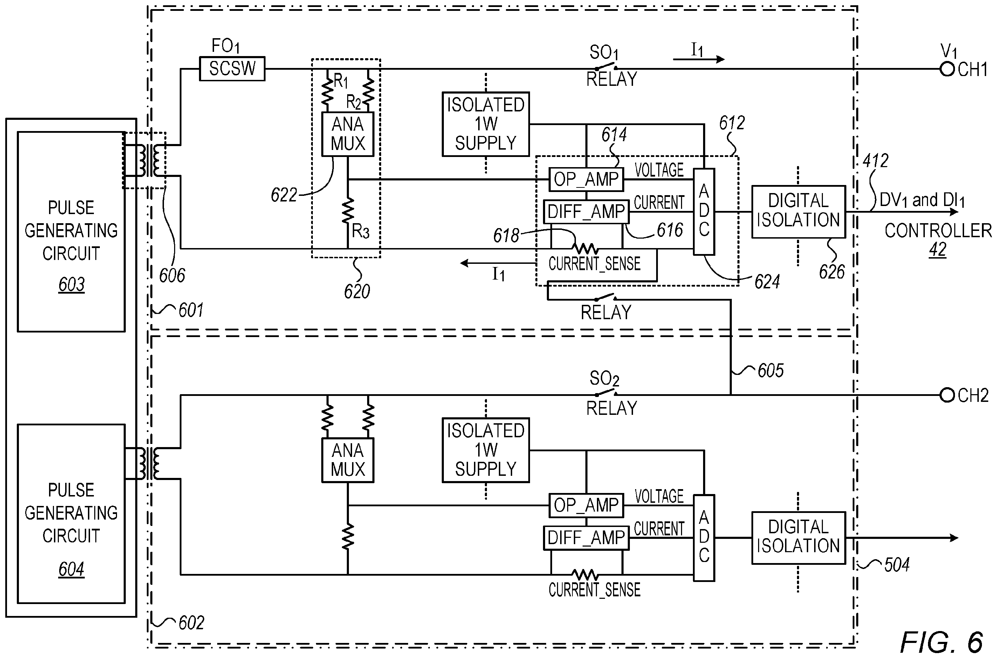

Fig. 5 is an electrical schematic diagram of pulse routing and metrology assembly 408 of Fig. 4, in accordance with an embodiment of the invention. For the sake of clarity, the circuits involved in measuring currents and voltages, have been omitted. These circuits will be detailed in Fig. 6, below. Output channels 422 and connection 424 are shown in Fig. 5 using the same labels as in Fig. 4.

Pulse routing and metrology assembly 408 comprises modules 502, with one module for each output channel 422. A pair 504 of adjacent modules 502 configured for bipolar IRE is shown in detail in Fig. 6, below. In the alternative, a BP line 506 connected to return patch 66 can be used as the return path for unipolar IRE. Modules 502 receive pulse inputs via respective transformer secondaries 508, 510, which are driven by primary coils in pulse generation assembly 406.

Each module 502 comprises switches and relays, labelled as FOᵢ, SOᵢ, Nᵢ, and BPᵢ for the i^{th} module. Switches FOᵢ are all fast switches, controlled by field-programmable gate arrays (FPGAs, not shown in the figure), for switching the IRE ablation from channel to channel, whereas switches SOᵢ, Nᵢ, and BPᵢ are slower relays, used to set up pulse routing and metrology assembly 408 for a given mode of IRE ablation. A typical switching time for fast switches FOᵢ is shorter than 0.3 µs, whereas slow relays SOᵢ, Nᵢ, and BPᵢ require a switching time of only 3 ms.

Fig. 6 is an electrical schematic diagram of two adjacent modules 601 and 602 of pulse routing and metrology assembly 408 configured for IRE in bipolar mode, in accordance with an embodiment of the invention. The use of module 601 for unipolar mode will be described further hereinbelow.

Modules 601 and 602 make up pair 504 of Fig. 5, as is shown by dash-dot frame with the same label (504). Modules 601 and 602 are fed by pulse generating circuits 603 and 604, respectively, which comprise, with reference to Fig. 4, parts of pulse generation assembly 406. Modules 601 and 602, in turn, feed channels CH1 and CH2, respectively, similarly to modules 502 of pair 504 in Fig. 5. Two modules 601 and 602 are shown in Fig. 6 in order to show a connection 605 between the modules. As the two modules are identical (and identical to the additional modules in pulse routing and metrology assembly 408), only module 601 is described in detail below.

Pulse generation assembly 406 comprises one pulse generating circuit similar to circuits 603 and 604 for each channel of IRE pulse generator 44. Pulse generating circuit 603 is coupled to module 601 by a transformer 606. Fast switch FO₁ and slow relays SO₁, N₁, and BP₁ are labelled similarly to Fig. 5.

A voltage V₁ and current I₁ coupled to CH1 are shown in Fig. 6 as a voltage between channels CH1 and CH2, and a current flowing to CH1 and returning from CH2.

V₁ and I₁ are measured by a metrology module 612, comprising an operational amplifier 614 for measuring the voltage and a differential amplifier 616 measuring the current across a current sense resistor 618. Voltage V₁ is measured from a voltage divider 620, comprising resistors R₁, R₂, and R₃, and an analog multiplexer 622. Analog multiplexer 622 couples in either resistor R₁ or R₂, so that the voltage dividing ratio of voltage divider 620 is either R₁/R₃ or R₂/R₃. Metrology module 612 further comprises an analog-to-digital converter (ADC) 624 for converting the measured analog voltage V₁ and current I₁ to digital signals DV₁ and DI₁. These digital signals are sent through a digital isolator 626 to controller 42 as signals 412 (Fig. 4). Controller 42 utilizes the received digital signals 412 for generating command signals 418 sent to pulse generation assembly 406 for adjusting the amplitudes and phases of the IRE pulses coupled to channels CH1 and CH2.

For the purpose of "virtual shorting," controller 42 receives signals 412 from respective modules 502. As an example of bipolar IRE with "virtually shorted electrodes," electrodes CH1, CH2, and CH3 (coupled to channels CH1, CH2, and CH3) are selected as one extended electrode and electrodes CH4, CH5, and CH6 are selected as the other extended electrode. The signals from electrodes CH1, CH2, and CH3 are coupled, after passing through tissue of patient 24, to channels CH4, CH5, and CH6 using relays shown in Fig. 5. Controller 42 receives from channels CH1, CH2, and CH3 signals 412 indicating the respective measured voltages and currents, and generates respective command signals 418 so that V₁, V₂, and V₃ each will have the same amplitude and phase (i.e., are virtually shorted). Similarly, controller 42 receives signals 412 from channels CH4, CH5, and CH6, and generates respective command signals 418 so that V₄, V₅, and V₆ each will have the same amplitude and phase, but differ from the amplitude and phase of V₁, V₂, and V₃ so that a desired train of IRE pulses flows between the two groups of channels (and consequently between the two groups of electrodes 34 coupled to these channels).

As an example of unipolar IRE with "virtually shorted electrodes," electrodes CH1, CH2, and CH3 are selected as one extended electrode, while return patch 66 functions as the return electrode for the IRE ablation signals emitted from electrodes CH1, CH2, and CH3. Return patch 66 is coupled via connection 424 to respective modules 502 of channels CH1, CH2, and CH3 by relays BP₁, BP₂, and BP₃. Similarly to bipolar IRE described above, controller 42 receives signals 412 from channels CH1, CH2, and CH3, and generates respective command signals 413 so that V₁, V₂, and V₃ each will have the same amplitude and phase, thus virtually shorting electrodes 34 coupled to these channels.

Digital isolator 626 protects patient 24 (Fig. 1) from unwanted electrical voltages and currents.

Switch FO₁, relays SO₁, BP₁, N₁ and 610, and analog multiplexer 622 are driven by controller 42. For the sake of simplicity, the respective control lines are not shown in Fig. 6.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art. The invention is defined by the appended claims.

## Claims

1. A medical apparatus (20) for irreversible electroporation, the medical apparatus comprising:
a probe (26), which comprises:
an insertion tube (28) configured for insertion into a body cavity of a patient; and
a distal assembly (30), which is connected distally to the insertion tube and comprises a plurality of electrodes (34), which are configured to contact tissue within the body cavity;
a common electrode (66) configured to be fixed to a location on the body of the patient;
an electrical signal generator (44), which is configured to generate biphasic electrical pulses and apply the biphasic electrical pulses simultaneously between a group of two or more of the electrodes and the common electrode in a unipolar mode so that the biphasic electrical pulses pass through the body from the group of electrodes to the common electrode with energy sufficient to irreversibly electroporate the tissue contacted by the electrodes in the group; and
a controller (42), which is coupled to measure time-varying voltage differences between the electrodes in the at least one group and to adjust the biphasic electrical pulses applied to the electrodes in the group so that the voltage differences do not exceed a predetermined threshold at any time during application of the biphasic electrical pulses.

2. The apparatus according to claim 1, wherein the controller is configured to adjust an amplitude of the biphasic electrical pulses so as to compensate for a difference in respective peak voltages measured at any pair of the electrodes of the group.

3. The apparatus according to claim 1, wherein the controller is configured to adjust a phase of the biphasic electrical pulses so as to compensate for a phase offset between respective voltage waveforms measured at any pair of the electrodes of the group.

4. A medical apparatus (20) for irreversible electroporation, the medical apparatus comprising:
a probe (26), which comprises:
an insertion tube (28) configured for insertion into a body cavity of a patient; and
a distal assembly (30), which is connected distally to the insertion tube and comprises a plurality of electrodes (34), which are configured to contact tissue within the body cavity;
an electrical signal generator (44), which is configured to generate biphasic electrical pulses and apply the biphasic electrical pulses simultaneously between a first group of two or more of the electrodes and a second group of two or more electrodes in a bipolar mode with energy sufficient to irreversibly electroporate the tissue contacted by the electrodes in the first and second groups; and
a controller (42),
wherein the controller is coupled to measure time-varying voltage differences between the electrodes in the first group and to adjust the biphasic electrical pulses applied to the electrodes in the first group so that the voltage differences do not exceed a predetermined threshold at any time during application of the biphasic electrical pulses, and
wherein the controller is coupled to measure time-varying voltage differences between the electrodes in the second group and to adjust the biphasic electrical pulses applied to the electrodes in the second group so that the voltage differences do not exceed a predetermined threshold at any time during application of the biphasic electrical pulses.

5. The apparatus according to claim 4, wherein the controller is configured to adjust an amplitude of the biphasic electrical pulses so as to compensate for a difference in respective peak voltages measured at any pair of the electrodes of the first group.

6. The apparatus according to claim 5, wherein the controller is configured to adjust an amplitude of the biphasic electrical pulses so as to compensate for a difference in respective peak voltages measured at any pair of the electrodes of the second group.

7. The apparatus according to any one of claims 4 to 6, wherein the controller is configured to adjust a phase of the biphasic electrical pulses so as to compensate for a phase offset between respective voltage waveforms measured at any pair of the electrodes of the first group.

8. The apparatus according to claim 7, wherein the controller is configured to adjust a phase of the biphasic electrical pulses so as to compensate for a phase offset between respective voltage waveforms measured at any pair of the electrodes of the second group.

9. The apparatus according to any previous claim, wherein the distal assembly comprises a balloon, which is connected distally to the insertion tube and is configured to be inflated within the body cavity with a fluid that flows into the balloon through the insertion tube.

## Patentansprüche

1. Medizinische Einrichtung (20) für die irreversible Elektroporation, die medizinische Einrichtung umfassend:
eine Sonde (26), die umfasst:
einen Einführschlauch (28), der für eine Einführung in eine Körperhöhle eines Patienten konfiguriert ist; und
eine distale Anordnung (30), die mit dem Einführschlauch distal verbunden ist und eine Vielzahl von Elektroden (34) umfasst, die konfiguriert ist, um Gewebe innerhalb der Körperhöhle zu kontaktieren;
eine gemeinsame Elektrode (66), die konfiguriert ist, um an einer Stelle auf dem Körper des Patienten befestigt zu werden;
einen elektrischen Signalgenerator (44), der konfiguriert ist, um biphasische elektrische Impulse zu generieren und die biphasischen elektrischen Impulse gleichzeitig zwischen einer Gruppe von zwei oder mehr der Elektroden und der gemeinsamen Elektrode in einem unipolaren Modus anzulegen, sodass die biphasischen elektrischen Impulse durch den Körper von der Gruppe von Elektroden zu der gemeinsamen Elektrode mit einer Energie fließen, die ausreicht, um das Gewebe irreversibel zu elektroporieren, das durch die Elektroden in der Gruppe kontaktiert wird; und
eine Steuerung (42), die gekoppelt ist, um zeitlich variierende Spannungsdifferenzen zwischen den Elektroden in der mindestens einen Gruppe zu messen und um die biphasischen elektrischen Impulse anzupassen, die an die Elektroden in der Gruppe angelegt werden, sodass die Spannungsdifferenzen zu keinem Zeitpunkt während einer Anlegung der biphasischen elektrischen Impulse einen vorbestimmten Schwellenwert überschreiten.

2. Einrichtung nach Anspruch 1, wobei die Steuerung konfiguriert ist, um eine Amplitude der biphasischen elektrischen Impulse anzupassen, um eine Differenz jeweiliger Spitzenspannungen auszugleichen, die an einem beliebigen Paar der Elektroden der Gruppe gemessen werden.

3. Einrichtung nach Anspruch 1, wobei die Steuerung konfiguriert ist, um eine Phase der biphasischen elektrischen Impulse anzupassen, um einen Phasenversatz zwischen jeweiligen Spannungswellenformen auszugleichen, die an einem beliebigen Paar der Elektroden der Gruppe gemessen werden.

4. Medizinische Einrichtung (20) für die irreversible Elektroporation, die medizinische Einrichtung umfassend:
eine Sonde (26), die umfasst:
einen Einführschlauch (28), der für eine Einführung in eine Körperhöhle eines Patienten konfiguriert ist; und
eine distale Anordnung (30), die mit dem Einführschlauch distal verbunden ist und eine Vielzahl von Elektroden (34) umfasst, die konfiguriert ist, um Gewebe innerhalb der Körperhöhle zu kontaktieren;
einen elektrischen Signalgenerator (44), der konfiguriert ist, um biphasische elektrische Impulse zu generieren und die biphasischen elektrischen Impulse gleichzeitig zwischen einer ersten Gruppe von zwei oder mehr der Elektroden und einer zweiten Gruppe von zwei oder mehr Elektroden in einem bipolaren Modus mit einer Energie anzulegen, die ausreicht, um das Gewebe irreversibel zu elektroporieren, das durch die Elektroden in der ersten und der zweiten Gruppe kontaktiert wird; und
eine Steuerung (42),
wobei die Steuerung gekoppelt ist, um zeitlich variierende Spannungsdifferenzen zwischen den Elektroden in der ersten Gruppe zu messen und um die biphasischen elektrischen Impulse anzupassen, die an die Elektroden in der ersten Gruppe angelegt werden, sodass die Spannungsdifferenzen zu keinem Zeitpunkt während der Anlegung der biphasischen elektrischen Impulse einen vorbestimmten Schwellenwert überschreiten, und
wobei die Steuerung gekoppelt ist, um zeitlich variierende Spannungsdifferenzen zwischen den Elektroden in der zweiten Gruppe zu messen und um die biphasischen elektrischen Impulse anzupassen, die an die Elektroden in der zweiten Gruppe angelegt werden, sodass die Spannungsdifferenzen zu keinem Zeitpunkt während der Anlegung der biphasischen elektrischen Impulse einen vorbestimmten Schwellenwert überschreiten.

5. Einrichtung nach Anspruch 4, wobei die Steuerung konfiguriert ist, um eine Amplitude der biphasischen elektrischen Impulse anzupassen, um eine Differenz der jeweiligen Spitzenspannungen auszugleichen, die an einem beliebigen Paar der Elektroden der ersten Gruppe gemessen werden.

6. Einrichtung nach Anspruch 5, wobei die Steuerung konfiguriert ist, um eine Amplitude der biphasischen elektrischen Impulse anzupassen, um eine Differenz der jeweiligen Spitzenspannungen auszugleichen, die an einem beliebigen Paar der Elektroden der zweiten Gruppe gemessen werden.

7. Einrichtung nach einem der Ansprüche 4 bis 6, wobei die Steuerung konfiguriert ist, um eine Phase der biphasischen elektrischen Impulse anzupassen, um einen Phasenversatz zwischen jeweiligen Spannungswellenformen auszugleichen, die an einem beliebigen Paar der Elektroden der ersten Gruppe gemessen werden.

8. Einrichtung nach Anspruch 7, wobei die Steuerung konfiguriert ist, um eine Phase der biphasischen elektrischen Impulse anzupassen, um einen Phasenversatz zwischen jeweiligen Spannungswellenformen auszugleichen, die an einem beliebigen Paar der Elektroden der zweiten Gruppe gemessen werden.

9. Einrichtung nach einem vorstehenden Anspruch, wobei die distale Anordnung einen Ballon umfasst, der mit dem Einführschlauch distal verbunden ist und konfiguriert ist, um innerhalb der Körperhöhle mit einem Fluid aufgeblasen zu werden, das durch den Einführschlauch in den Ballon fließt.

## Revendications

1. Appareil médical (20) destiné à une électroporation irréversible, l'appareil médical comprenant :
une sonde (26), qui comprend :
un tube d'insertion (28) conçu pour insertion dans une cavité corporelle d'un patient ; et
un ensemble distal (30), qui est relié de manière distale au tube d'insertion et comprend une pluralité d'électrodes (34), qui sont conçues pour venir en contact avec du tissu à l'intérieur de la cavité corporelle ;
une électrode commune (66) conçue pour être fixée à une localisation sur le corps du patient ;
un générateur de signaux électriques (44), qui est configuré pour générer des impulsions électriques biphasées et appliquer les impulsions électriques biphasées simultanément entre un groupe de deux des électrodes ou plus et l'électrode commune dans un mode unipolaire de sorte que les impulsions électriques biphasées traversent le corps à partir du groupe d'électrodes vers l'électrode commune avec une énergie suffisante pour réaliser une électroporation irréversible du tissu en contact avec les électrodes dans le groupe ; et
une unité de commande (42), qui est couplée pour mesurer des différences de tension à variation temporelle entre les électrodes dans l'au moins un groupe et pour ajuster les impulsions électriques biphasées appliquées aux électrodes dans le groupe de sorte que les différences de tension ne dépassent pas un seuil prédéterminé à n'importe quel moment pendant l'application des impulsions électriques biphasées.

2. Appareil selon la revendication 1, dans lequel l'unité de commande est configurée pour ajuster une amplitude des impulsions électriques biphasées de façon à compenser une différence dans les tensions de crête respectives mesurées au niveau de l'une quelconque paire des électrodes du groupe.

3. Appareil selon la revendication 1, dans lequel l'unité de commande est configurée pour ajuster une phase des impulsions électriques biphasées de façon à compenser un déphasage entre les formes d'ondes de tension respectives mesurées au niveau de l'une quelconque paire des électrodes du groupe.

4. Appareil médical (20) destiné à une électroporation irréversible, l'appareil médical comprenant :
une sonde (26), qui comprend :
un tube d'insertion (28) conçu pour insertion dans une cavité corporelle d'un patient ; et
un ensemble distal (30), qui est relié de manière distale au tube d'insertion et comprend une pluralité d'électrodes (34), qui sont conçues pour venir en contact avec du tissu à l'intérieur de la cavité corporelle ;
un générateur de signaux électriques (44), qui est configuré pour générer des impulsions électriques biphasées et appliquer les impulsions électriques biphasées simultanément entre un premier groupe de deux des électrodes ou plus et un second groupe de deux électrodes ou plus dans un mode bipolaire avec une énergie suffisante pour réaliser une électroporation irréversible du tissu en contact avec les électrodes dans les premier et second groupes ; et
une unité de commande (42),
dans lequel l'unité de commande est couplée pour mesurer des différences de tension à variation temporelle entre les électrodes dans le premier groupe et pour ajuster les impulsions électriques biphasées appliquées aux électrodes dans le premier groupe de sorte que les différences de tension ne dépassent pas un seuil prédéterminé à n'importe quel moment pendant l'application des impulsions électriques biphasées, et
dans lequel l'unité de commande est couplée pour mesurer des différences de tension à variation temporelle entre les électrodes dans le second groupe et pour ajuster les impulsions électriques biphasées appliquées aux électrodes dans le second groupe de sorte que les différences de tension ne dépassent pas un seuil prédéterminé à n'importe quel moment pendant l'application des impulsions électriques biphasées.

5. Appareil selon la revendication 4, dans lequel l'unité de commande est configurée pour ajuster une amplitude des impulsions électriques biphasées de façon à compenser une différence dans les tensions de crête respectives mesurées au niveau de l'une quelconque paire des électrodes du premier groupe.

6. Appareil selon la revendication 5, dans lequel l'unité de commande est configurée pour ajuster une amplitude des impulsions électriques biphasées de façon à compenser une différence dans les tensions de crête respectives mesurées au niveau de l'une quelconque paire des électrodes du second groupe.

7. Appareil selon l'une quelconque des revendications 4 à 6, dans lequel l'unité de commande est configurée pour ajuster une phase des impulsions électriques biphasées de façon à compenser un déphasage entre les formes d'ondes de tension respectives mesurées au niveau de l'une quelconque paire des électrodes du premier groupe.

8. Appareil selon la revendication 7, dans lequel l'unité de commande est configurée pour ajuster une phase des impulsions électriques biphasées de façon à compenser un déphasage entre les formes d'ondes de tension respectives mesurées au niveau de l'une quelconque paire des électrodes du second groupe.

9. Appareil selon l'une quelconque revendication précédente, dans lequel l'ensemble distal comprend un ballonnet, qui est raccordé distalement au tube d'insertion et est conçu pour être gonflé au sein de la cavité corporelle avec un fluide qui s'écoule dans le ballonnet à travers le tube d'insertion.
